# EUROPEAN PATENT SPECIFICATION

(11) **EP 1 482 915 B1**
(45) Date of publication and mention of the grant of the patent: **13.01.2010**
(21) Application number: 03732266.6
(22) Date of filing: 12.03.2003
(51) Int. Cl.: A61K 9/28

(54) **THERAPEUTIC SYSTEM FOR THE CONTROLLED RELEASE OF ACTIVE INGREDIENTS**
THERAPEUTISCHES SYSTEM FÜR DIE KONTROLLIERTE FREISETZUNG VON WIRKSTOFFEN
SYSTEME THERAPEUTIQUE DE LIBERATION CONTROLEE D'INGREDIENTS ACTIFS

(30) Priority: 12.03.2002 IT MI20020514
(43) Date of publication of application: 08.12.2004
(73) Proprietor: JAGOTEC AG, 4132 Muttenz (CH)
(72) Inventor: MAGGI, Lauretta, I-27100 Pavia (IT); CONTE, Ubaldo, I-21052 Busto Arsizio (IT)
(74) Representative: Bassil, Nicholas Charles
(86) International application number: PCT/EP2003/002536
(87) International publication number: WO 2003/075897

(56) References cited:
- EP-A- 0 274 734
- EP-A- 0 631 775
- WO-A-94/01239

## Description

### STATE OF THE ART

In the last decades great importance has surrounded the research for the optimisation of the release of active substances from matrices and/or other systems containing them, so that the activity is carried out in the place and at the time desired. This problem finds many fields of application but is above all relevant in the pharmaceutical sector both for human and veterinary use. It is relevant in the agricultural sector too, for example for the use of fertilisers, herbicides or selective pesticides.

In the above indicated sectors, there is an enormous amount of research and development attempting to guarantee the achievement of the release of active substances at controlled rate, independently of the environmental factors in which the matrix or the system containing the active substance is found.

In the biomedical sector, there have been great advances, in the research and the developments of forms of administration or therapeutic systems able to give up the active substance vehicularised in them at a constant rate and for a period of time pre-determinable *in vitro,* with the aim of obtaining identical or analogous behaviour *in vivo.*

The aim is that of achieving a system able to reach, and maintain over time, predetermined plasma concentrations of active substances; such systems are classified as systems having a release kinetics defined as "zero order".

The research in this sector has examined all the administration routes destined for human therapy, but surely the sector in which there has been the most activity is that of the oral administration systems; this is due to the importance, both in terms of numbers and economics, of the pharmaceutical forms for oral administration in the global market, also due to the high degree with which oral administration is used among patients and to the undoubted advantages of stability showed by the active substances when vehicularised in a solid pharmaceutical form such as a tablet or a more sophisticated and complex therapeutic system, but however in the solid state.

Amongst the innovative, complex pharmaceutical forms for oral administration, the therapeutic systems defined as osmotic pumps, usually Indicated as OROS systems can be cited (US patent No. 4,160,020); which In fact refer to a system capable of releasing the drug at constant rate over time.

The search for systems which are less complex and more manageable and economical to produce, has brought about the preparation and the commercialisation of matrix based systems of particular geometry, such as those claimed in the US patents No. 5,422,123 and No. 5,738,874. Other therapeutic systems designed for constant rate of release of the active ingredient are described in "Novel Drug Delivery" by Prescott and Nimmo (J. Wiley - New York 1989), in "Controlled Release of drugs: Polymers and Aggregate Systems" M. Rosoff Ed. (VCH Pub. New York 1989) and in "Controlled release dosage form design" Chemg-ju Kim Ed. (Technomic - Lancaster 2000).

Above all in the treatment of chronic diseases, which in the majority of cases involve elderly patients, the pharmaceutical forms and the embodiments described above have the undoubted advantage of allowing a drastic simplification of the dosage schemes with administrations which, in many_cases, can be reduced to once a day, obtaining in addition a greater observance (compliance) by the patient of the prescribed therapy.

The administration of constant release, pharmaceutical forms is particularly important if the active ingredient has an efficacious therapeutic effect when plasma levels are achieved and maintained over time which are defined in a precise "range"; below of which values the drug is inactive, whilst at higher values are seen manifestations and side effects and/or toxic phenomena which, in some cases can be extremely dangerous.

It is however worth noting that not always and not for every disease is it necessary and appropriate to achieve and maintain constant plasma levels of drug, in fact in certain diseases and in some symptomologies with disease manifestations bound to chrono-biological rhythms it is favourable to have pharmaceutical forms able to release the active ingredient in successive, impulses so as to prevent giving rise to worse pain or pathological manifestations bound, as said above, to circadian rhythms.

Studies have in fact underlined that many functions of the cardiovascular, respiratory, renal and hepatic systems have important variations over the course of the day; the plasma concentrations of many hormones such as insulin, cortisol, adrenaline, aldosterone, angiotensin and other substances such as glucose, plasma proteins and enzymes also follow circadian rhythms. In addition the symptoms and the attacks of some diseases are not manifested in a casual manner throughout the day. For example, asthma attacks are more frequent during the night, myocardial infarction is manifested with higher probability in the late morning, angina pectoris attacks are more frequent in the early hours of the morning, acute arthritis attacks are verified with greater frequency in the early morning as with the tremors typical of Parkinson's disease and the symptoms of psychiatric disorders.

With such symptomologies the need for the availability of pharmaceutical forms or therapeutic systems able to release the active ingredient or different active ingredients in a pulsed manner, appears evident, i.e. to release doses of different drug(s) over time at programmable time intervals.

An example of a system of pulsed release is that reported in the European patent No. 0274734 in which is described a three layered tablet of which two are coated with a sheathing container constituted of impermeable polymer material and insoluble in water or soluble in alkaline environments. In the patented description and in the examples accompanying the patent are reported the preparation of a three layered tablet of which the first and the third vehicularise the active ingredient, whilst the layer intermediate between them, is constituted of gelable polymeric material. The sheath which coats the second and the third layers and allows the immediate release of a first dose of drug, whilst the second portion is released after a time interval of approx. 30 minutes characterises the system. The system described in the above cited patent has however a dramatic limitation in use, because the application of the coating is carried out manually and each tablet is singularly subjected to the partial coating procedure through particularly long procedures, not easily standardisable and however not transferable to the industrial scale.

Analogous limitations to the industrial feasibility are present in other embodiments described in the European patent application No. 0788790 in which a nucleus with a partial external coating is disclosed, in which said nucleus is of three layers of which the upper layer contains a dose of active substance, the intermediate layer is constituted of polymeric material with the function of a retardant barrier and the lower layer contains the remaining dose of active substance. The external coating is constituted of polymeric materials applied by compression with a complex procedure and not easily industrially applicable. A further improvement of the system described in the European patent application No. 0788790 and reported in the patents US No. 5,487,901 and US No. 5,650,169 in which is described a three layered tablet completely coated by a film of impermeable polymeric material a part of which is eliminated by an abrasion process: with the abrasion of the coating was also abraded and eliminated a part of the layer which contains the active substance thus giving rise to a possibly inferior active substance content to the minimal limit necessary for the therapy or however a loss of active substance.

### SUMMARY OF INVENTION

A new programmed release therapeutic system has now been found constituted of a three layered tablet completely coated by a film insoluble in aqueous fluids on which incisions have been made by laser beams which delimit one or more surfaces of predetermined shape and size, removable when the therapeutic system comes into contact with aqueous fluids, releasing the corresponding surface(s) of the tablet. With the system according to the present invention after the release of the first dose, the latency time for the release of the second dose of the same or a different active ingredient can be programmed in a precise interval of time, from 15 minutes to 6-10 hours.

The fact that the procedure for the preparation of the above mentioned therapeutic system uses production technologies of use widely consolidated and such as to allow the regulated and standardised preparations of the pulsed release pharmaceutical form, in particular the laser technology is particularly important.

### BRIEF DESCRIPTION OF THE DRAWINGS

Figure 1: cross sectional view of a rounded shape three-layered tablet showing the outer layers (1) and (2) comprising the active ingredient(s), and the inner barrier layer (3), completely coated by the film coating (4), on which one or more incisions has to be made.
Figure 2: cross sectional view of the tablet in Figure 1, showing one incision (5) only on a face of the coating.
Figure 3: cross sectional view of the tablet in Figure 1, showing two incisions (5) on the faces of the coating.

### DETAILED DESCRIPTION OF THE INVENTION

The present invention refers to release system comprising a tablet or nucleus comprising at least three completely overlying layers, completely coated with a film insoluble in aqueous fluid. Of these layers which constitute the tablet (nucleus):
- the first layer contains one or more active substance formulated so as to be released rapidly or in a gradual manner;
- the second layer, the barrier layer, is formulated so as to constitute a "barrier" to the release of the active substance vehicularised in the third layer. Such barrier layer has fundamentally the role of rendering independent the release of the first and the third layer. Furthermore it is able to slowly interact (in a time interval programmable by *in vitro* tests) with the means of dissolution (aqueous and/or characterised by different pH values), therefore protecting the third layer for a predeterminable period of time from contact with the means of dissolution until said means of dissolution penetrates through the aperture in the coating of the first layer;
the third layer contains one or more active substance, identical or different to that contained in the first layer, formulated in a manner such as to be released rapidly or slowly both if placed directly in contact with the aqueous fluid, and after the erosion of the barrier layer, which separates the third layer from the first.

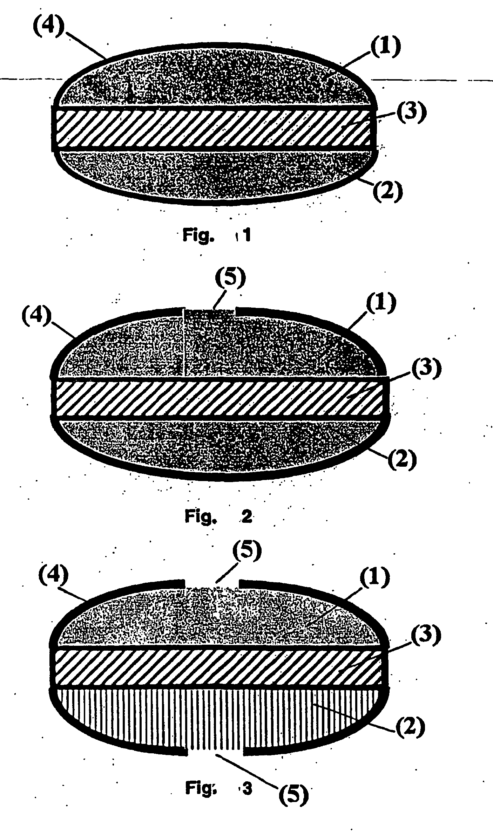

In the preferred embodiment the tablet is as schematically reported in Figure 1, i.e. of rounded shape.

The first and the third layer, which contain the active substance, can be identical or different compositions and thickness. The thickness of the two layers vary depending on the active substances and of the other components vehicularised in it.

The above described tablet of the invention is completely and uniformly coated by a filming procedure in a shallow basin or by another industrially applicable procedure, with appropriate coatings which impede the release of the active ingredient. Preferably, such coating films are insoluble and impermeable to aqueous liquids in the pH interval of between 1.2 to 9. A fundamental characteristic of the system of the invention is the fact that the insoluble film coating is etched by a laser beam of appropriate power and intensity, applied for an interval of time determined depending on the characteristics and the thickness of the coating. The laser beam creates, in the coating, one or more incisions which delimit a surface of geometrical shape and of an area predetermined with extreme precision. It is possible to make such incisions with precision such that only the insoluble coating is notched without interfering with the underlying tablet. The contact of the new release system with water or with biological fluid determines the infiltration of the fluid through the incision(s) and therefore the beginning of the erosion or the slow gelation of the constituents of the matrix system of the nucleus with the consequent raising of the parts of the film around the incision(s). The portion of insoluble coating delimited by the incision(s) breaks off allowing the interaction between the means of dissolution and the nucleus containing the active substance through the surfaces of the nucleus remaining uncovered. The coating, in addition, remains intact until the moment of use and is partially removed only *in vivo,* so that it protects the active ingredients contained in the tablet from humidity and oxidation, thus increasing the stability and conferring to the tablet an optimal mechanical resistance.

In any case, the interaction with the means of dissolution and the release of the active ingredient can take place only through the hole made in the coating, at a rate which depends on the area exposed and is therefore exactly predeterminable. The therapeutic release system according to the present invention is extremely flexible and allows a pulsed release of active ingredients according to various alternatives which allow to satisfy the most different dosage needs.

The incision can delimit an area of said coating of dimensions ranging between 2 and 50% and preferably between 5 and 30% of the total area of the coating. According to a first embodiment of the invention the incision(s) in the coating are made on the face of the tablet which is found corresponding to the first layer. When the system is used, the interaction of the nucleus with the means of dissolution - and therefore the release of the active substance vehicularised in the first layer - can take place only through the surface devoid of coating. Upon contact with water or with biological fluids the first dose of drug begins to be released through the hole(s) made in the coating, at a rate which depends on the area exposed and therefore, in the case in which the hole(s) are circular, on the diameter of the hole(s). A the end of the programmed release of the first dose, the presence of the barrier layer impedes the release of the second dose of drug as long as the barrier itself will not have been eroded or disaggregated by the means of dissolution. Therefore the characteristics of the barrier, and in particular the necessary time for its erosion or disaggregation, determine the duration of the time interval between the release of the first dose of drug and of the second dose. If the active ingredient is the same in the first and in the third layer one will have the pulsed release of a single drug with a time interval between the first and second delivery exactly programmable. The embodiment described above is schematically represented in Figure 2, which shows a tablet or nucleus with a portion of the surface corresponding to the incision in the coating corresponding with the first layer immediately available for contact with aqueous liquids and therefore with the active ingredient contained in said rapidly releasable layer, whilst all the remaining portion of the tablet (corresponding to the second layer-barrier and to the third layer) is homogeneously and regularly coated.

According to a further embodiment of the invention it is possible to have different active ingredients in the first and in the third layer and the incision of the soluble coating layer made only in correspondence with the first layer of the tablet (nucleus). With such a system the administration of two different drugs is possible, with a predetermined time interval and with a release passage which can be regulated by the composition of the first and of the third layer which contains the two different active ingredients and which can be different.

According to a further embodiment of the invention it is possible to have different active ingredients in the first and in the third layer and cut with the laser the insoluble coating film, both in correspondence with the first and with the third layer. In such a case it is possible to administer simultaneously two different active ingredients with different release kinetics which can be regulated both as a function of the composition of the layer and as a function of the shape and dimension(s) of the apertures made in the coating by laser incision (see Figure 3). According to a further embodiment of the present invention on the filmed tablet which has one or more incisions a second gastroresistant and enterosoluble polymeric coating is applied. This allows a further control of the release because in a gastric environment the active substance vehicularised in the tablet is not released and the system activates only at the enteric level, when the enterosoluble coating undergoes solubilisation. The release of the vehicularised active ingredient begins only following such solubilisation and will be only through the hole(s) made in the coating of impermeable polymeric material. This configuration of the tablet allows the active substance to be released only at the enteric level and can be used to obtain the release of the drug up to the distal portion of the enteric tract, for a release at the level of the colon or the rectum. Schematically the composition which constitutes the nucleus of the release system according to the invention can be thus described:

### First layer

If a rapid release of the active substance is desired, the composition of the first layer comprises polymeric substances capable of favouring the disintegration of said layer, thus facilitating the dissolution and the rapid release of the active ingredients vehicularised in it. For example, said polymeric substances can be selected from the group consisting of cross linked polyvinylpyrrolidone, sodium amidoglycolate, carboxymethylcellulose, salts and derivatives thereof, cross linked sodium carboxymethylcellulose, polyoxyethylene, carboxymethylamide, hydroxypropylcellulose and low and medium molecular weight hydroxypropylmethylcellulose, potassium methacrylate-divinylbenzene co-polymer, polyvinylalcohols, glucan, scleroglucan, mannan, starches, starch derivatives, microcrystalline cellulose and cellulose derivatives, beta cyclodextrin and dextrin derivatives in general. As far as hydroxypropylmethylcellulose is concerned, various types can be used with different molecular weights (from 1000 to 4000000) and with different degrees of substitution. These types of hydroxypropylmethylcellulose have different characteristics and may be prevalently erodible or prevalently gelable, as a function of the degree of substitution (D.S.) in the polymeric chain. Also, as far as the polyoxyethylenes are concerned, various types with different molecular weights (from 1000 to 4000000) can be used and with different properties: they can be prevalently erodible or prevalently gelable, as a function of the molecular weight of the polymeric chain.

The polymeric substances constitute from 1 to 90 % of the weight of the layer, preferably from 5% to 60%.

In addition the so called effervescent mixtures can be used, capable that is of producing the rapid disintegration of the tablet or, in the specific case, of the layer when it comes into contact with aqueous liquids or with gastric juices.

These effervescent mixtures may comprise mixtures constituted of acids such as citric, tartaric, fumaric acids and carbonates and bicarbonates of sodium and other alkaline or alkaline earth metals, glycocoll, sodium carbonate and other pharmaceutically acceptable salts, capable of determining the appearance of the "effervescence" and the rapid disintegration of the compacted matter.

Other coadjuvant substances, selected from the group comprising substances normally used in the pharmaceutical field, such as diluents, buffers, binding agents, adsorbents etc. can also be used, and in particular starch, pre-gelatinised starch, calcium phosphate, mannitol, lactose, sucrose, glucose, xylitol, sorbitol, microcrystalline cellulose and binding agents such as gelatine, polyvinylpyrrolidone, methylcellulose, starch indicator, ethylcellulose, gum arabic, and tragacanth gum.

In addition, other excipients normally used in pharmaceutical techniques can be used as coadjuvants such as magnesium stearate, calcium stearate, sodium fumarate, stearic acid, talc, colloidal silica, glyceryl monostearate, polyethylene glycols of molecular weights from 200 to 20000, hydrogenated castor oil, waxes and mono-di- and tri- substituted glycerides.

If instead a slow release of the active substance contained in the first layer is desired, excipients able to slow down the hydration and disintegration and/or to favour the slow erosion of said layer can find use, slowing down in such a manner the dissolution and the release of the first dose of active substance contained in said layer. In the examples attached to the present application will be better highlighted the characteristics of said layer.

The thickness of the first layer ranges between 0.5 and 5.0 mm.

### Second layer (barrier)

In the case of the configuration of the system as described in Figure 2, the formulation of the "barrier" layer - which allows the determination of the time interval necessary for the liberation of the active ingredient contained within the third layer - comprises polymeric substances, preferably in association with coadjuvants and plasticising substances.

The polymeric substances of the barrier layer can be selected, for example, from the group consisting of hydroxypropylmethylcellulose with molecular weight ranging between 1000 and 4000000, hydroxypropylcellulose with molecular weights ranging between 2000 and 2000000, polyoxyethylenes (PEO) with molecular weights ranging between 1000 and 10000000, carboxyvinyl polymers, polyvinyl alcohols with molecular weights ranging between 10000 and 1000000, polyamides, polyanhydrides, polyvinylpyrrolidone, glucans, scleroglucans, mannans, xanthans, carragenans, galactomannans, gellans, polyaminoacids, poly(methyl vinyl ethers/ maleic anhydride), carboxymethylcellulose and derivatives, ethylcellulose, methylcellulose, cellulose derivatives in general, alginic acid and salts and derivatives thereof, starches, starch derivatives, α, β- γ-cyclodextrins and copolymers of the above mentioned polymers.

The polymeric substances are present in percentages of between 5 to 90% with respect to the total weight of said layer and preferably from 30% to 90%.

The coadjuvant substances can be selected, for example, from the group consisting of glyceryl monostearate and semi synthetic triglyceride derivatives, semi-synthetic glycerides, hydrogenated castor oil, glycerylpalmitoylstearate, glyceryl beenate, cetyl alcohol, polyvinylpyrrolidone, ethylcellulose, methylcellulose, sodium carboxymethylcellulose, and other natural or synthetic substances well known to all the experts in the art. For example magnesium stearate, stearic acid, talc, sodium benzoate, boric acid, polyethyleneglycols and colloidal silica are used.

In addition, diluents, binding agents, lubricants, buffers, antiadherents, glidants and other substances capable of conferring the desired characteristics to said layer can be used, as will become better explained in the examples outlined below. Also, other pharmaceutically acceptable substances capable of favouring the compressibility of the mixture and/or of optimising the production process can find use; such substances have the function of conferring onto the barrier layer the necessary elasticity and of improving the adhesion, cohesion and resistance characteristics.

The coadjuvant substances, in association with the previously reported polymeric materials, are capable of better defining the period of "holding" of the barrier, allowing such interval to vary from 15 minutes up to 6-10 hours according to the required therapeutic necessity.

The barrier layer, as reported above and as will be underlined better in the examples accompanying the present invention, can be constituted of polymers predominantly erodible and/or soluble in water and in aqueous liquids.

When however, the therapeutic requirements require that the second dose of drug or, however, that the active substance contained in the third layer must be released slowly and in a gradual manner, after a determined period of time from the release of the first dosage, then said barrier layer will be preferably constituted of predominantly gelable polymers. Operating thusly, it is possible to obtain a rapid release of the first dose of drug and, following a defined time interval, the release of the second dose which, proceeding gradually and at a rate programmable in vitro, guarantees the maintenance of efficacious plasma levels of the drug. Analogously, if the system of the invention has a configuration as reported in Figure 3, the barrier layer can be predominantly constituted of hydrophilic and gelable polymeric materials, the barrier in this case having the function of separating the first and third layers which .contain different active substances and which can be incompatible with each other.

The thickness of said second layer (barrier) is comprised of between 0.5 and 5.0 mm.

### Third layer

The third layer can be constituted of an identical or different formulation from that adopted in the first layer. In addition, the third layer can contain identical active ingredient(s) or can vehicularise different active ingredient(s): in this case the system has a configuration as reported in Figure 2, Alternatively, the third layer can vehicularise one or more active ingredients different from these vehicularised in the first layer, giving the system a configuration as reported in Figure 3. In this case, the active ingredient(s) of the third layer is released simultaneously with the active ingredients contained in the first layer at a rate which will depend on the composition of the layer itself.

The thickness of said third layer is comprised of between 0.5 and 5.0 mm.

### Coating

The fundamental characteristics of the system described is constituted by the fact that all the three layered tablets are completely coated, according to procedures known in the art (in shallow basins, in fluidised beds or with other procedures), with a layer (film) of polymeric material which is impermeable to water or to aqueous fluids, at least for a period of time such as to allow both the release of the first dose of active ingredient and the dissolution of the second barrier layer prior to the release of the second dose of active ingredient.

Such filming agents, for example, ethylcellulose (of different molecular weights) and acrylic and methacrylic polymers can find use.

According to an embodiment of the present invention onto the filmed three layered tablet which has one or more incisions, in the insoluble coating, as described above, can also be applied a second gastroresistant and enterosoluble polymeric coating. Such a coating can be constituted for example of acrylic and methacrylic copolymers, cellulose acetate-phthalate, cellulose acetate-propionate, cellulose trimellitate and other natural synthetic or semi-synthetic cellulose derivatives, of hydroxypropylcellulose, of hydroxypropylmethylcellulose, for example hydroxypropylmethylcellulose acetate succinate.

In the filming operation of the three layered tablet coadjuvants such as colouring agents, opacifying and plasticising substances such as triethylcitrate, diethylphthalate, butyl phthalate, diethylsebacate, polyoxyethyleneglycols of molecular weights ranging between 300 and 50000 can find use.

The thickness of the coating film may range from 5 to 1000 µm but preferably from 20 to 500 µm.

The claimed system is characterised by the fact that the insoluble coating film has one or more incisions delimiting an area of appropriately calculated geometric shape and dimensions.

The incision in the coating film can be carried out on only one face of the system (as schematically represented in Figure 2) so as to allow, as already said, contact of the means of dissolution *in vitro* or of biological fluids *in vivo* only with the first layer containing the first medication.

Alternatively, the incision in the coating film can be carried out on both faces of the system (as schematically represented in Figure 3) so as to allow contact with the means of dissolution simultaneously with the active ingredients contained in the first and third layers.

To carry out the incision operation in the coating film a laser beam of appropriate energy and intensity is used; it is applied for an interval of time defined on the basis of the characteristics and the thickness of said film, as will be better explained in the following Examples. Preferably the incision in the coating film is performed using a CO₂ source laser apparatus having a power of 20 W.

The laser beam provokes a precise incision in the coating film delimiting an area of geometrical shape (a circle in the most simple case) and of predetermined dimensions in function to the flow of drug desired per unit of time.

Operating as indicated above one can obtain filmed tablets, coated all over the surfaces with the exception of one or two portions of the surfaces exactly defined and measurable, as schematically reported in Figures 2 and 3, for non limiting illustration of the invention. That means that all the surfaces of the tablet are impermeable to aqueous liquids with the exclusion of the only surface devoid of coating. In fact, the incision in the coating film allows the penetration into the nucleus of the dissolving liquid. The contact with aqueous liquid or with biological liquids allows the activation of the system and begins the release of the active substance(s) vehicularised in the system.

Amongst the active substances of possible use in the present system can be listed all the active substances able to carry out their curative and/or protective action towards the disease manifestations which are manifested according to temporal and in particular to circadian rhythms, for example: non steroid anti inflammatory substances (NSAID) such as sodium diclofenac, indomethacin, ibuprofen, ketoprofen, diflunisal, pyroxicam, naproxene, flurbiprofen, sodium tolmethin, paracetamol, anti inflammatory steroids or sleep inducing substances and tranquillisers such as diazepam, nitrazepam, flurazepam, oxazepam, chlordiazepoxide, medazepam, lorazepam, active ingredients for the control of hypertension such as amlodipine, captopril, clonidine, dilthiazem, enalapril, felodipine, katanserine, lisinopril, methyldopa, nifedipine, nitrendipine, nicardipine, prazosine, ramipril, beta blockers such as atenolol, metoprolol, pindolol, propanolol, timolol, diuretics such as amiloride, clortalidone, frusemide, hydrochlorotiazide, indapamide, spironolattone, anti Parkinson's drugs such as amantidine, bromocriptin, levodopa, antihistamines such as tripelennamine, terfenadine, anti asthmatics such as ketothiophene, nedocromil, antibiotics alone and in association or chemotherapeutic agents. The tablets of the invention can be produced, starting from mixtures of powders and/or granulates, using the current production technologies and therefore with production processes immediately scalable to the industrial level.

For example, they can be produced using rotary pressing machines suitable for producing "multi-layered" tablets such as for example Elisabeth Hata, Korsch or Manesty Layer-press. Normally, the working pressure varies from 1000 to 5000 kg, obtaining according to the procedure better detailed in the following Examples, three layered tablets spheroid, ovoid in shape and, however, without notable spikes, such as to allow said pharmaceutical forms to be subjected to the successive filming process using known technologies such as coating in shallow basins or in fluidised beds.

The following examples are reported as non limiting illustrations of the present invention.

Example 1: The preparation of a series of 5000 three layered tablets as reported in Figure 2, containing as the active ingredients in the first and third layer diltiazem (two doses of 60 mg each) and an intermediate barrier layer.

### 1.a Preparation of the granulate containing the active ingredient

| | |
|---|---|
| Diltiazem (Profarmaco -Milan) | 60.0 mg |
| Corn starch (USP grade, C Erba, Milan, I) | 30.0 mg |
| Lactose (USP grade, C Erba, Milan, I) | 40.0 mg |
| Methylcellulose (Methocel^{®} A4- Colorcon - U.K) | 0.2 mg |
| Polyvinylpyrrolidone (cross linked)(Polyplasdone ISP-Wayne, NY, USA) | 10.0 mg |
| Sodium carboxymethylamide (Explotab^{®} - E. Mendell USA) | 10.0 mg |
| Magnesium stearate (C Erba, Milan, I) | 1.0 mg |
| Colloidal silicate (Syloid^{®} 244, Grace GmbH, Worms, D | 0.5 mg |
| Total | 151.7mg |

The envisaged quantity of diltiazem is mixed, in an appropriate mixer, with the lactose and the com starch; the homogeneous mixture obtained is wetted with an aqueous solution of 1.3% methylcellulose in water. The uniformly humidified mass is forced through a grid of 25 mesh (equal to 710 microns) obtaining a granulate which is dried in a hot air circulating oven until a constant weight. The dried granulate, is re-calibrated through the same grille, has the disaggregants, the magnesium stearate and the colloidal silicate added. The mass is mixed in a V shaped mixer for 30 minutes.

The granulate thus obtained is used for the preparation of the first and the third layer, as will be described in detail in the following point 1.c.

### 1.b. Preparation of the granulate for the production of the barrier layer

The granulate has the following percentage composition:

| | |
|---|---|
| Hydroxypropylmethylcellulose (Methocel^{®} E5 Colorcon -U.K.) | 75.5% |
| Hydrogenated castor oil (Cutina^{®} HR - Henkel -D) | 18.8% |
| Polyvinylpyrrolidone (Povidone ISP-Wayne, NY, USA) | 2.8 % |
| Blue laquer (Colorcon^{®} -U.K) | 0.1 % |
| Magnesium stearate (C Erba, Milan, I) | 1.9 % |
| Colloidal silica (Syloid^{®} 244, Grace GmbH,Worms, D | 0.9 % |
| Total | 100 % |

The envisaged amount of hydroxypropylmethylcellulose and hydrogenated castor oil are mixed, in an appropriate mixer, with the blue laquer colouring; the homogeneous mixture obtained, lightly blue coloured, is humidified in a hydroalcoholic solution of 10% polyvinylpyrrolidone. The uniformly humid mass is forced through a 25 mesh grille (equal to 710 µm) obtaining a granulate which is dried in a hot air circulating oven until constant weight. The dried granulate, re-calibrated through the same grid, is added with magnesium stearate and colloidal silicate. The mass is mixed in a V shaped mixer for 30 minutes. The granulate thus obtained is used for the preparation of the second layer, as will be described in detail in the following example 1.c.

### 1.c - Preparation of the three layered systems (by compression).

The granules obtained according to such procedures outlined and according to schemes well known to any person skilled in the art, are loaded into the three loading hoppers of a rotary press suitable for producing three layered tablets (e.g. Manesty Layer-Press, Liverpool, UK). In particular into the first and third hopper is loaded the granulate described in point 1.a; whilst in the second hopper is loaded the granulate according to that described in the preceding example 1.b.

The pressing machine is equipped with rounded circular dies of 10.0 mm diameter and a radius of curvature of 12 mm; thus allowing the attainment of circular rounded tablets.

The machine is set up so as to produce three layered overlaid systems constituted respectively of 151.7 mg of granulate containing the active ingredient (equal to 60 mg of diltiazem) 150.0 mg of granulate prepared in example 1.b (such a quantity being necessary to obtain a layer of approx. 1.5 mm in thickness) and a second dose of 151.7 mg of granulate containing the active ingredient.

Operating as previously described, three layered tablets having a mean weight of 453.4 mg, containing two distinct doses of 60 mg active ingredient, are obtained.

### 1.d -Filming coating

The tablets thus obtained are subjected to filming in a basin using an aqueous coating dispersion the percentage composition of which w/w is described as follows.

### Copolymer of acrylic and methacrylic acid

| | |
|---|---|
| (Eudragit^{®} L 30 D - Rohm - Pharma -D) | 17.5 % |
| Talc(C. Erba, Milan, I) | 5.3 % |
| Triethylcitrate (C. Erba. Milan, I) | 1.7 % |
| Titanium dioxide (C. Erba, Milano, I) | 5.3 % |
| Water | 70.2 % |
| Total | 100 % |

The filming operation is carried out in a traditional stainless steel basin 30 cm in diameter; the solution of the polymeric coating material is sprayed with a traditional air jet system (Asturo Mec type with nozzles from 1.0 mm). The filming operation is carried out until the application of a continuous, homogeneous and regular coating film is achieved for each tablet with a thickness of approx. 100 µm.

Operating according to the above described procedure, completely coated three layered tablets, as highlighted in Figure 1, are obtained.

### 1.e - Incisions of the film coating (with circular incisions of 5.0 mm in diameter delimiting a surface coating of 19.6 mm²).

The filmed tablets are placed in an appropriate vibrator-distributor which orient and distribute the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular on the upper face of the filmed tablet is performed a circular incision of 5.0 mm in diameter.

The incision is carried out in a time of around 100 milliseconds, an application necessary and sufficient to perforate the coating film of a thickness of about 100 µm.

Operating, as described above filmed tablets coated in every part of the surface are obtained, with the exception of the surfaces cut by laser beam as described in Figure 2. That means that all the coated surfaces of the tablet are impermeable to aqueous liquids with the exclusion of the cut surfaces.

### 1.f - Dissolution test

To evaluate the release characteristics of the finished system, described in example 1.e (the circular incision of the film of 5.0 mm in diameter) is used the apparatus 2, paddle (described in USP XXII) operating at 100 r.p.m. and using such a dissolution fluid as 0.1 N hydrochloric acid at 37°C. The release of the active ingredient is followed by UV spectrophotometric determination at 236 nm using an automatic sampling and reading system (Spectracomp 602 from Advanced Products- Milan)

The results of the test carried out are reported in table I

**TABLE I**

| Time (min) | % release |
|---|---|
| 15 | 30.2 |
| 30 | 48.7 |
| 60 | 50.8 |
| 120 | 51.0 |
| 240 | 51.3 |
| 360 | 51.5 |
| 480 | 52.8 |
| 600 | 98.7 |
| 720 | 100.9 |

From the results highlighted in the Table above, it is evident that a rapid release of the first dose (50% of the total dose contained in the system) occurs within 30-60 minutes, followed by an interval of around 9 hours during which drug is not released, and then release of the second dose of active ingredient following 10 hours from the beginning of the dissolution test. Such behaviour fully answers the objectives of the present invention.

Example 2: Preparation of a series of 5000 filmed three layered tablets as described in Fig. 2, containing as the active ingredient in the first and third layer diltiazem (two doses of 60 mg each) and an intermediate barrier layer.

The preparation of the filmed tablets is carried out using the procedure described in example 1 up to point 1.d. The peculiarity of Example 2 lies in the different dimensions of the coating surface delimited by the incisions.

### 2.e - Incision of the film coat (with a circular incision of 7.0 mm in diameter delimiting a coating surface of 38.5 mm²)

The filmed tablets are placed in an appropriate vibrator-distributor which orients and distributes the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular on the upper face of the filmed tablet is performed a circular incision of 7.0 mm in diameter.

The incision is performed in a time of around 100 milliseconds, the application necessary and sufficient to perforate the coating film with a thickness of approx. 100 µm.

Operating as described above, filmed tablets coated on every part of their surfaces are obtained, with the exception of the surfaces cut by the laser beam as described in Figure 2. That means that all the coated surfaces of the tablet are impermeable to aqueous liquids with the exception of the cut surfaces.

### 2.f - Dissolution test.

To evaluate the release characteristics of the finished systems, described in example 2.e (with a circular abrasion of 7.0 mm in diameter) is used the apparatus 2, paddle (described in USP XXII) operating at 100 r.p.m. and using 0.1 N hydrochloric acid at 37°C as such dissolution fluid. The release of the active ingredient is followed by UV spectrophotometric determination at 236 nm using an automatic sampling and reading system (Spectracomp 602 from Advanced Products, Milan).

The results of the test performed are reported in Table II

**TABLE II**

| Time (min) | % released |
|---|---|
| 15 | 39.6 |
| 30 | 51.1 |
| 60 | 52.0 |
| 120 | 52.6 |
| 240 | 53.3 |
| 360 | 68.5 |
| 420 | 95.8 |
| 480 | 101.3 |

From the results in the Table above, it is evident that a rapid release of the first dose (50% of the total dose contained within the system) is obtained in 15-30 minutes, followed by an interval of around 5-6 hours during which drug is not released and by the release of the second dose of the active ingredient after around 6-7 hours from the beginning of the dissolution test. Such behaviour fully answers the objectives of the present invention.

Example 3: The preparation of a series of 5,000 filmed three layered tablets as described in Fig. 2, containing as the active ingredient in the first and third layer diltiazem (two doses of 60 mg each) and an intermediate barrier layer.

The preparation of the filmed tablets is carried out using the procedure described in example 1 up to point 1.d. The peculiarity of Example 3 lies in the different dimensions of the coating surface delimited by the incisions.

### 3.e - Incision of the film coating (with circular incisions of 9.0 mm in diameter delimiting 63.6 mm² of surface)

The filmed tablets are placed in an appropriate vibrator-distributor which orients and distributes the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular, on the upper face of the filmed tablet is performed a circular incision of 9 mm in diameter equal to 63.6 mm² of surface.

The incision is performed in a time of around 100 milliseconds, the application necessary and sufficient to perforate the coating film of a thickness of approx. 100 µm.

Operating as described above, filmed tablets coated on every part of their surfaces are obtained, with the exception of the surfaces cut as described in the Figure 2. That means that all the coated surfaces of the tablet are impermeable to aqueous liquids with the exception of the cut surfaces.

### 3.f - Dissolution test

To evaluate the release characteristics of the finished systems, described in Example 3.e (with circular incisions of 9.0 mm in diameter) is used the apparatus 2. paddle (described in USP XXII) operating at 100 r.p.m. and using 0.1 N hydrochloric acid at 37°C as such dissolution fluid. The release of the active ingredient is followed by UV spectrophotometric determination at 236 nm using an automatic sampling and reading system (Spectracomp 602 from Advanced Products, Milan).

The results of the tests carried out are reported in table III

**TABLE III**

| Time (min) | % release |
|---|---|
| 5 | 23.0 |
| 15 | 40.6 |
| 30 | 50.9 |
| 120 | 51.2 |
| 180 | 72.7 |
| 240 | 97.0 |
| 300 | 99.8 |

From the results in the Table above, it is evident that a rapid release of the first dose (50% of the total dose contained within the system) occurs in 15-30 minutes, followed by an interval of around 2.5-3 hours during which no drug is released and by the release of the second dose of the active ingredient after approx. 4 hours from the start of the dissolution test. Such behaviour fully answers the objectives of the present invention.

Example 4: The preparation of a series of 5000 three layered tablets as described in Fig. 2, containing such active ingredient in the first and third layer diltiazem (the first dose of 50% and the second dose of 50%) and an intermediate barrier layer. The barrier layer differs with respect to the previous examples in that hydroxypropylmethylcellulose (Methocel^{®} E3) having lower molecular weight and viscosity is used.

### 4.a - Preparation of the granulate containing the active ingredient

| | |
|---|---|
| Diltiazem (Profarmaco -Milan) | 60.0 mg |
| Corn starch (USP grade, C Erba, Milan, I) | 30.0 mg |
| Lactose (USP grade, C Erba, Milan, I) | 40.0 mg |
| Methylcellulose (Methocel^{®} A4- Colorcon - U.K) | 0.2 mg |
| Polyvinylpyrrolidone (cross linked) (Polyplasdone ISP-Wayne, NY, USA) | 10.0 mg |
| Sodium carboxymethylamide (Explotab^{®} - E. Mendell USA) | 10.0 mg |
| Magnesium stearate (C Erba, Milan, I) 1.0 mg | |
| Colloidal silicate (Syloid^{®} 244, Grace GmbH,Worms, D | 0.5 mg |
| Total | 151.7 mg |

The envisaged amount of diltiazem is mixed, in a appropriate mixer, with the lactose and the corn starch; the homogeneous mixture obtained is humidified with an aqueous solution of 1.3% methylcellulose in water. The uniformly humid mass is forced through a 25 mesh grid (equal to 710 µm) obtaining a granulate which is dried in a hot air circulating oven until constant weight. The dried granulate, re-calibrated through the same grille, is added to the disaggregants, the magnesium stearate and the colloidal silicate. The mass is mixed in a V shaped mixer for 30 minutes.

The granulate thus obtained is used for the preparation of the first and third layer, as will be described in detail in the following example 4.c.

### 4.b - Preparation of the granulate for the production of the second layer (barrier layer)

The granulate has the following percentage compositions:

| | |
|---|---|
| hydroxypropylmethylcellulose (Methocel^{®} E3 Colorcon^{®} -U.K.) | 76.5 % |
| Hydrogenated castor oil (Cutina^{®} HR - Henkel -D) | 19.0% |
| Polyvinylpyrrolidone (Povidone ISP-Wayne, NY, USA) | 2.9 % |
| Green laquer (Aluminium oxide) | 0.1% |
| Stearic acid (C Erba, Milan, I) | 1.5 % |
| Total | 100.0 % |

The envisaged amount of hydroxypropylmethylcellulose and of hydrogenated castor oil is mixed, in an appropriate mixer, with the green coloured laquer; the homogeneous mixture obtained, lightly green in colour, is humidified with an hydroalcoholic solution of 10% Polyvinylpyrrolidone. The uniformly humidified mass is forced through a 25 mesh grid (equal to 710 µm) obtaining a granulate which is dried in a hot air circulation oven until constant weight. The dried granulate, re-calibrated through the same grille, is added to the stearic acid. The mass is mixed in a V shaped mixer for 30 minutes.

The granulate thus obtained is used for the preparation of the second layer, as will be described in detail in the following example 4.c.

### 4.c - Preparation of the three layered systems (by compression).

The granulates obtained according to the above described procedures and well known in the art, are loaded into three loading hoppers of a rotary press suitable for producing three layered tablets (e.g. Manesty Layer-Press, Liverpool, UK). In particular in the first and third hopper are loaded the granulate described in Example 4.a; whilst in the second hopper is loaded the granulate according to the procedure described in the preceding Example 4.b.

The machine press is equipped with rounded circular dies of 10.0 mm of diameter and a radius of curvature of 12 mm, thus allowing the attainment of rounded circular tablets.

The machine is set up such as to produce three layer systems constituted respectively of 151.7 mg of granulate containing the active ingredient (equal to 60 mg of diltiazem), 50.0 mg of the granulate prepared in Example 4.b (such a quantity being necessary to obtain a barrier layer with a thickness of 1.5 mm approx.) and of 151.7 mg of the granulate containing the active ingredient (equal to 60 mg of diltiazem).

Operating as previously described, three layered tablets of mean weight of 453.4 mg containing two distinct doses of the active ingredient of 60 mg each, are obtained.

### 4.d - Coating for filming.

The tablets thus obtained are subjected to filming in basins using an aqueous coating dispersion the percentage composition of which w/w is reported as follows.

| | |
|---|---|
| Dispersion of ethylcellulose (Surelease^{®} clear Colorcon^{®} U.K.) | 60.0 % |
| water | 40.0 % |
| Total | 100.0% |

The filming operation is performed in a traditional stainless steel basin 30 cm in diameter, the dispersion of polymeric coating material is constituted of ethylcellulose and contains such diethylsebacate plasticising agents and oleic acid as stabiliser, the dispersion, diluted in water prior to use, is sprayed with a traditional air jet system (Asturo Mec type with nozzle from 1.0 mm). The filming operation is carried out until the application of a continuous, homogeneous and regular film coating for each tablet.

One obtains, operating in the described manner completely coated three layered tablets, as highlighted in Figure 1.

### 4.e - Incisions in the film (with circular incisions of 7.0 mm in diameter delimiting a surface of 38.5 mm²)

The filmed tablets are placed in an appropriate vibrator-distributor which orients and distributes the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular, on the upper face of the filmed tablet is performed a circular incision of 7.0 mm in diameter.

The incision is performed in a time of around 100 milliseconds, such application necessary and sufficient to perforate the coating film of a thickness of around 100 µm.

Operating as described above filmed tablets coated on every part of the surface are obtained, with the exception of the cut surfaces as described in Figure 2. That means that all the coated surfaces of the tablets are impermeable to aqueous liquids with the exception of the cut surfaces.

### 4.f - Dissolution test.

To evaluate the release characteristics of the finished systems, described in 4.e (a circular incision of 7.0 mm in diameter) is used the apparatus 2, paddle (described in USP XXII) operating at 100 r.p.m. and using 0.1 N hydrochloric acid at 37°C as such dissolution fluid. The release of the active ingredient is followed by UV spectrophotometric determination at 236 nm using an automatic sampling and reading system (Spectracomp 602 from Advanced Products- Milan)

The results of the tests carried out are reported in Table IV

**TABLE IV**

| Time (min) | % release |
|---|---|
| 15 | 21.4 |
| 30 | 48.3 |
| 60 | 51.1 |
| 120 | 51.8 |
| 180 | 52.0 |
| 210 | 75.8 |
| 240 | 97.3 |
| 300 | 101.9 |

From the Table above, it is evident that a rapid release of the first dose (50% of the total dose contained within the system) occurs in 15-30 minutes, followed by an interval of around 2.5 hours during which no drug is released and by the successive release of the second dose of active ingredient following approx. 4 from the start of the dissolution test. Such behaviour fully answers the objectives of the present invention.

Example 5: The preparation of a series of 5000 three layered tablets as reported in Fig. 3, comprising such active ingredient in the first layer 50 mg of hydrochlorothiazide, in the third layer 80 mg of propanolol and a barrier layer.

### 5.a - Preparation of the granulate containing 50 mg of hydrochlorothiazide

| | |
|---|---|
| Hydrochlorothiazide (Profarmaco -Milan) | 50.0 mg |
| Hydroxypropylmethylcellulose (Methocel^{®} E 15LV - Colorcon^{®}) | 40,0 mg |
| Lactose (USP grade, C Erba, Milan, I) | 40.0 mg |
| Methylcellulose (Methocel^{®} A4- Colorcon^{®} - U.K) | 0.4 mg |
| Magnesium stearate (C Erba, Milan, I) | 1.0 mg |
| Colloidal silica (Syloid^{®} 244, Grace GmbH,Worms, D | 0.5 mg |
| Total | 131.9 mg |

The envisaged amount of hydrochlorothiazide is mixed, in appropriate mixer, with hydroxypropylmethylcellulose and lactose; the homogeneous mixture obtained is humidified with an aqueous solution of 1.3% methylcellulose in water. The uniformly humidified mass is forced through a 25 mesh grid (equal to 710 µm) obtaining a granulate which is dried in a hot air circulating oven until constant weight. The dried granulate, re-calibrated through the same grid, is added to the magnesium stearate and the colloidal silica. The mass is mixed in a V shaped mixer for 30 minutes.

The granulate thus obtained is used for the preparation of the first layer, as will be described in detail in the following point 1.c.

### 5.b - Preparation of a granulate containing 80 mg of propanolol

| | |
|---|---|
| -Propranolol (Sigma -Milan) | 80.0 mg |
| Hydroxypropylmethylcellulose (Methocel^{®} E 50 LV - Colorcon^{®}) | 50.0 mg |
| Lactose (USP grade, C Erba, Milan, I) | 50.0 mg |
| Blue laquer (Colorcon^{®} - U.K) | 1.0 mg |
| Methylcellulose (Methocel^{®} A4- Colorcon^{®} - U.K) | 0.4 mg |
| Magnesium stearate (C Erba, Milan, i) | 1.0 mg |
| Colloidal silica (Syloid^{®} 244, Grace GmbH,Worms, D | 0.5 mg |
| Total | 182.9 mg |

The envisaged amount of propanolol is mixed, in appropriate mixer, with hydroxypropylmethylcellulose and lactose and blue laquer, the homogeneous mixture obtained, lightly green in colour, is humidified with an aqueous solution of 1.3% methylcellulose in water. The uniformly humid mass is forced through a 25 mesh grid (equal to 710 µm) obtaining a granulate which is dried in a hot air circulating oven until constant weight. The dried granulate, re-calibrated through the same grille, is added to the magnesium stearate and the colloidal silica. The mass is mixed in a V shaped mixture for 30 minutes. The granulate thus obtained is used for the preparation of the third layer, as will be described in detail in the following example 5.d.

### 5.c - Preparation of the granulate for the production of the second layer (barrier layer)

The granulate has the following percentage composition:

| | |
|---|---|
| Hydroxypropylmethylcellulose (Methocel^{®} E 50 LV - Colorcon^{®} -U.K.) | 76.5 % |
| Hydrogenated castor oil (Cutina^{®} HR - Henkel -D) | 19.0 % |
| Polyvinylpyrrolidone (Povidone ISP-Wayne, NY, USA) | 2.9 % |
| Orange laquer (Colorcon^{®} - U.K) | 0.1 % |
| Stearic acid (C Erba, Milan, I) | 1.5% |
| Total | 100.0 % |

The envisaged amount of hydroxypropylmethylcellulose and hydrogenated castor oil are mixed, in an appropriate mixer, with the orange coloured laquer; the homogeneous mixture obtained, lightly coloured, is humidified with a hydroalcoholic solution of 10% polyvinylpyrroridone. The uniformly humidified mass is forced through a 25 mesh grid (equal to 710 µm) obtaining a granulate which is dried in a hot air circulating oven to constant weight. The dried granulate, re-calibrated through the same grille, is added to the stearic acid. The mass is mixed in a V shaped mixer for 30 minutes.

The granulate thus obtained is used for the preparation of the second layer, as will be described in detail in the following point 5.d.

### 5.d - Preparation of the three layered systems (by compression).

The granulates obtained according to the above described procedures well known to any person skilled in the art, are loaded into three loading hoppers of a rotary press suitable to produce three layered tablets (e.g. Manesty Layer-Press, Liverpool, UK). In particular in the first and third hopper is loaded the granulate containing the hydrochlorothiazide, described in point 5.a; whilst in the second hopper is loaded the granulate constituting the intermediate barrier, described in the preceding example 5.c and in the third hopper is loaded the granulate containing the propanolol, described in example 5.b. The machine press is fitted with rounded circular dies of 10.0 mm in diameter and radius of curvature = 12 mm, allowing thus to obtain rounded circular tablets.

The machine is set up so as to produce three layered systems constituted respectively of 131.9 mg of granulate containing 50 mg of hydrochlorothiazide, 150.0 mg of the granulate prepared in the example 5.c (such quantity being necessary to obtain a thickness of 1.5 mm approx.) and 182.9 mg of the granulate containing 80 mg of propanolol.

Operating as described above, three layered tablets of mean weight of 464.8 mg containing 50 mg of hydrochlorothiazide and 80 mg of propanolol, are obtained.

### 5.e - Coating for filming.

The tablets thus obtained are subjected to film coating in a basin using an ethanolic solution of ethylcellulose using diethylphthalate as plasticizer.

The filming operation is carried out in a traditional stainless steel basin of 30 cm in diameter; the solution of polymeric coating material is sprayed with a traditional air jet system (Asturo Mec type with a nozzle of 1.0 mm). The filming operation is carried out until the application of a continuous, homogeneous and regular coating film is obtained for each tablet.

Operating in the described manner, completely coated three layered tablets are obtained, as highlighted in Figure 1.

### 5.f - Incision of the film coat (with circular incisions of 5.0 mm in diameter equal to 19.6 mm2) on both faces of the tablet.

The filmed tablets are placed in an appropriate vibrator-distributor which orients and distributes the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular on both faces of the filmed tablet are performed circular incisions of 5.0 mm in diameter equal to 19.6 mm².

The incisions are carried out in a time of approx. 100 milliseconds, the application necessary and sufficient to perforate the coating film with a thickness of approx. 100 µm.

Operating as reported above, filmed tablets coated on every part of the surface are obtained, with the exception of the cut surfaces as described in Figure 3. That means that all the coated surfaces of the tablet are impermeable to aqueous liquids with the exception of the surfaces on which the film has been removed.

### 5.g- Dissolution test. (On filmed tablets a circular incision of 5.0 mm in diameter) To evaluate the release characteristics of the finished systems, outlined in 5.f is used the apparatus 2, paddle (described in USP XXII) operating at 100 r.p.m. and using distilled water at 37°C as such dissolution fluid. The release of the active ingredient is followed by spectrophotometric determination at 270 nm for the hydrochlorothiazide and at 290 nm for the propanolol, using an automatic sampling and reading system (Spectracomp 602 from Advanced Products- Milan)

The results of the tests performed are reported in the following Table V.

**TABLE V**

| Time (min) | % release | % release |
|---|---|---|
| | hydrochlorothiazide | propanolol |
| 0 | 0 | 0 |
| 120 | 10.9 | 12.3 |
| 240 | 23.2 | 25.1 |
| 360 | 37.3 | 38.4 |
| 720 | 62.0 | 68.2 |
| 960 | 72.4 | 82.5 |
| 1200 | 81.2 | 92.7 |
| 1440 | 88.8 | 96.9 |

From the Table above, a release of hydrochlorothiazide and of propanolol in approx. 24 hours occurs, with a more or less linear kinetics for both the active ingredients.

Such behaviour completely answers the objectives of the present invention.

Example 6: Preparation of a series of 5000 filmed tablets, with three layers as described in Fig. 3, comprising as the active ingredient in the first layer 50 mg of hydrochlorothiazide, in the third layer 80 mg of propanolol and an intermediate barrier layer.

The difference with respect to Example 5 consists in the greater surface of the film delimited by the incisions (38.5 mm² of surface). The preparation of the filmed tablets is carried out exactly as described in Examples 5.a to 5.e.

### 6 - f Incision of the film coating (with a circular incision of 7.0 mm in diameter equal to an area of 38.5 mm²) on both faces of the tablet

The filmed tablets are placed in an appropriate vibrator-distributor which serves to orient and distribute the tablets singularly on suitably precise housings with calibrated dimensions. A transport system allows the carrying of the single tablets positioned on the maximal stability surface under the ablation system constituted of a CO₂ laser beam source with a power rating of 20 W which carries out the removal of a portion of the film. In particular on both faces of the filmed tablet are performed a circular incision of 7.0 mm in diameter.

The incision is carried out in a time of approx. 100 milliseconds, the application necessary and sufficient to perforate the coating film with a thickness of approx. 100 µm.

Operating as described above, filmed tablets coated on every part of the surface are obtained, with the exception of the surfaces cut as described in Figure 3. That means that all the coated surface of the tablet is impermeable to aqueous liquids with the exception of the cut surfaces.

### 6.g- Dissolution test. (On filmed tablets with circular incisions of 7.0 mm in diameter)

To evaluate the release characteristics of the finished systems, described in Example 6-f is used the apparatus 2, paddle (described in USP XXII) operating at 100 r.p.m. and using distilled water at 37°C as such dissolution fluid. The release of the active ingredient is followed by UV spectrophotometric determination at 270 nm for the hydrochlorothiazide and at 290 nm for the propanolol, using an automatic sampling and reading system (Spectracomp 602 from Advanced Products- Milan).

The results of the tests carried out are reported in the following Table VI.

**TABLE VI**

| Time (min) | % release | % release |
|---|---|---|
| | Hydrochlorothiazide | propanolol |
| 0 | 0 | 0 |
| 120 | 15.3 | 21.6 |
| 240 | 35.8 | 36.9 |
| 360 | 56.4 | 54.3 |
| 720 | 77.0 | 86.3 |
| 960 | 85.3 | 96.2 |
| 1200 | 92.8 | 98.8 |
| 1440 | 99.8 | 99.8 |

From the Table above, it comes out that a release of hydrochlorothiazide and propanolol occurs with a more or less linear kinetics for both the active ingredients. In addition the release of the active ingredients is notably faster with respect to tablets which have free circular surfaces of 5.0 mm in diameter equal to 19.6 mm² of free surface on each face of the tablet.

Such behaviour completely answers the objectives of the present invention.

## Claims

1. Therapeutic system for the controlled release of one or more active ingredients, with previously programmed passage, **characterised by** the fact that it comprises a nucleus constituted of a three layered tablet of which the two external layers vehicularise the active ingredient(s) whilst the internal layer is constituted of a polymeric barrier erodible or gelable in aqueous means, said tablet being completely coated by a film of polymeric material insoluble in aqueous fluids, on which coating only have been carried out by laser one or more incisions which do not interfere with the underlying tablet and which delimit an area of geometric shape and predetermined dimensions as a function of the release times which it is desired to obtain, said release taking place from the area of the nucleus underlying the surfaces of the film coating delimited by the incisions, which are detached when the therapeutic system comes into contact with aqueous fluids:

2. Therapeutic system according to claim 1, in which the coating film is incised only in correspondence with the first layer of the tablet (nucleus).

3. Therapeutic system according to claim 1, in which the film coating is incised in correspondence both with the first and the third layer.

4. Therapeutic system according to claim 2, in which both the first and the third layer comprise the same active ingredient.

5. Therapeutic system according to claim 2, in which the first and the third layer comprise different active ingredients.

6. Therapeutic system according to claim 3, in which the first and the third layer comprise different active ingredients.

7. Therapeutic system according to claim 1, in which the first and the third layer have an identical composition for the controlled release of the active ingredient.

8. Therapeutic system according to claim 1, in which the first and third layer have different compositions for the controlled release of the active ingredient.

9. Therapeutic system according to claim 1, in which the area delimited by the incision(s) on the insoluble coating film has dimensions comprised of between 2 and 50% of the total area of the coating.

10. Therapeutic system according to claim 9, in which the area delimited by the incision(s) on the insoluble coating film is of dimensions comprised of between 5 and 30% of the total area of the coating.

11. Therapeutic system according to claim 1, in which the first layer comprises one or more polymers able to modulate the release of the active ingredient.

12. Therapeutic system according to claim 11, in which said polymers constitute between 1% and 90% in weight of said layer.

13. Therapeutic system according to claim 12, in which said polymers constitute between 5% and 60% in weight of said layer.

14. Therapeutic system according to claim 1, in which the first layer comprises excipients capable of accelerating the release of the active ingredient(s).

15. Therapeutic system according to claim 14, in which said excipients are disintegrants or effervescent mixtures.

16. Therapeutic system according to claim 1, in which the third layer comprises one or more polymers capable of modulating the release of the active ingredient(s).

17. Therapeutic system according to claim 16, in which said polymers constitute between 1% and 90% in weight of said layer.

18. Therapeutic system according to claim 17, in which said polymers constitute between 5% and 60% in weight of said layer.

19. Therapeutic system according to claim 1, in which the third layer comprises excipients able to accelerate the release of the active ingredient.

20. Therapeutic system according to claim 19, in which said excipients are disaggregants or effervescent mixtures.

21. Therapeutic system according to claim 1, in which the second layer comprises one or more polymers selected from predominantly erodible polymers and predominantly gelifyable polymers.

22. Therapeutic system according to claim 21, in which said polymers constitute between 5 and 90% in weight of said layer.

23. Therapeutic system according to claim 22, in which said polymers constitute from 30 to 90% in weight of said layer.

24. Therapeutic system according to claim 1, in which said three layers each have a thickness comprised of between 0.5 and 5 mm.

25. Therapeutic system according to claim 1, in which on the insoluble polymeric coating film a second gastroresistant and enterosoluble polymeric coating film is applied.

26. Process for the preparation of the therapeutic system according to claim 1, **characterised by** the fact that the incision(s) on the coating film are performed by the use of a laser beam.

27. Process according to claim 26, in which the incision(s) on the coating film are performed with a CO₂ source laser device having an output of 20 W.

## Patentansprüche

1. Therapeutisches System fur die kontrollierte Freisetzung von einem oder mehreren Wirkstoffen, mit vorhergehend programmiertem Durchlass, **gekennzeichnet durch** die Tatsache, dass es einen Kern, gebildet aus einer dreischichtigen Tablette umfasst, von welcher die beiden außeren Schichten als Vehikel fur den/die Inhaltsstoff(e) fungieren, wahrend die innere Schicht aus einer polymeren Barriere gebildet ist, welche in wasserigen Mitteln erodierfähig oder gelierfahig ist, wobei besagte Tablette vollstandig **durch** einen Film aus polymerem Material überzogen ist, welches in wasserigen Flüssigkeiten unlöslich ist, auf welchem Überzug nur **durch** Laser ein oder mehrere Einschnitte durchgefuhrt worden sind, welche nicht in die darunter liegende Tablette eingreifen, und welche einen Bereich von geometrischer Form und vorbestimmten Ausmaßen als eine Funktion der Freisetzungszeiten, welche wunschenswert zu erhalten ist, abgrenzen, wobei besagte Freisetzung aus dem Bereich des Kerns stattfindet, welcher unter den Oberflachen des Filmuberzugs, abgegrenzt **durch** die Einschnitte liegt, welche abgebaut werden, wenn das therapeutische System in Kontakt mit wässerigen Flüssigkeiten kommt.

2. Therapeutisches System gemaß Anspruch 1, in welchem der Überzugsfilm nur in Ubereinstimmung mit der ersten Schicht der Tablette (Kern) eingeschnitten ist.

3. Therapeutisches System gemaß Anspruch 1, in welchem der Filmüberzug in Übereinstimmung mit sowohl der ersten, als auch der dritten Schicht eingeschnitten ist.

4. Therapeutisches System gemaß Anspruch 2, in welchem sowohl die erste, als auch die dritte Schicht den gleichen Wirkstoff umfassen.

5. Therapeutisches System gemaß Anspruch 2, in welchem die erste und die dritte Schicht verschiedene Wirkstoffe umfassen.

6. Therapeutisches System gemaß Anspruch 3, in welchem die erste und die dritte Schicht verschiedene Wirkstoffe umfassen.

7. Therapeutisches System gemaß Anspruch 1, in welchem die erste und die dritte Schicht eine identische Zusammensetzung fur die kontrollierte Freisetzung des Wirkstoffs haben.

8. Therapeutisches System gemaß Anspruch 1, in welchem die erste und dritte Schicht verschiedene Zusammensetzungen fur die kontrollierte Freisetzung des Wirkstoffs haben.

9. Therapeutisches System gemaß Anspruch 1, in welchem der durch den/die Einschnitt(e) abgegrenzte Bereich auf dem unloslichen Uberzugsfilm Ausmaße hat, welche zwischen 2 und 50% des Gesamtbereichs des Uberzugs umfassen.

10. Therapeutisches System gemaß Anspruch 9, in welchem der durch den/die Einschnitt(e) abgegrenzte Bereich auf dem unloslichen Uberzugsfilm Ausmaße hat, welche zwischen 5 und 30% des Gesamtbereichs des Uberzugs umfassen.

11. Therapeutisches System gemaß Anspruch 1, in welchem die erste Schicht ein oder mehrere Polymere umfasst, welche in der Lage sind, die Freisetzung des Wirkstoffs zu modulieren.

12. Therapeutisches System gemäß Anspruch 11, in welchem besagte Polymere zwischen 1 und 90 Gew.-% der besagten Schicht bilden.

13. Therapeutisches System gemaß Anspruch 12, in welchem besagte Polymere zwischen 5 und 60 Gew.-% der besagten Schicht bilden.

14. Therapeutisches System gemaß Anspruch 1, in welchem die erste Schicht Arzneimitteltrager umfasst, welche in der Lage sind, die Freisetzung des Wirkstoffs/der Wirkstoffe zu beschleunigen.

15. Therapeutisches System gemaß Anspruch 14, in welchem besagte Arzneimitteltrager Abbaumittel oder aufschaumende Gemische sind.

16. Therapeutisches System gemaß Anspruch 1, in welchem die dritte Schicht ein oder mehrere Polymere umfasst, welche in der Lage sind, die Freisetzung des Wirkstoffs/der Wirkstoffe zu modulieren.

17. Therapeutisches System gemaß Anspruch 16, in welchem besagte Polymere zwischen 1 und 90 Gew.-% von besagter Schicht bilden.

18. Therapeutisches System gemaß Anspruch 17, in welchem besagte Polymere zwischen 5 und 60 Gew.-% von besagter Schicht bilden.

19. Therapeutisches System gemaß Anspruch 1, in welchem die dritte Schicht Arzneimitteltrager umfasst, welche in der Lage sind, die Freisetzung des Wirkstoffs zu beschleunigen.

20. Therapeutisches System gemaß Anspruch 19, in welchem besagte Arzneimitteltrager Abbaumittel oder aufschaumende Gemische sind.

21. Therapeutisches System gemaß Anspruch 1, in welchem die zweite Schicht ein oder mehrere Polymere umfasst, ausgewahlt aus uberwiegend erodierfahigen Polymeren und uberwiegend gelierfähigen Polymeren.

22. Therapeutisches System gemaß Anspruch 21, in welchem besagte Polymere zwischen 5 und 90 Gew.-% der besagten Schicht bilden.

23. Therapeutisches System gemaß Anspruch 22, in welchem besagte Polymere von 30 bis 90 Gew.-% der besagten Schicht bilden.

24. Therapeutisches System gemaß Anspruch 1, in welchem besagte drei Schichten jeweils eine Dicke haben, welche zwischen 0,5 und 5 mm umfasst.

25. Therapeutisches System gemaß Anspruch 1, in welchem auf dem unlöslichen polymeren Uberzugsfilm ein zweiter magenresistenter und enteroloslicher polymerer Uberzugsfilm aufgetragen ist.

26. Verfahren fur die Herstellung des therapeutischen Systems gemaß Anspruch 1, **gekennzeichnet durch** die Tatsache, dass der/die Einschnitt(e) auf dem Uberzugsfilm **durch** die Verwendung eines Laserstrahls durchgefuhrt werden.

27. Verfahren gemaß Anspruch 26, in welchem der/die Einschnitt(e) auf dem Uberzugsfilm mit einer CO₂-Quelle-Laservorrichtung mit einer Arbeitsleistung von 20 W durchgefuhrt werden.

## Revendications

1. Système thérapeutique pour la libération contrôlée d'un ou de plusieurs ingrédients actifs, comprenant un passage préalablement programmé, **caractérisé par le fait qu'**il comprend un noyau constitué d'un comprimé à trois couches parmi lesquelles les deux couches extérieures véhiculent l'ingrédient (les ingrédients) actif(s) tandis que la couche intérieure est constituée d'une barrière polymère pouvant être érodée ou gélifiée dans un milieu aqueux, ledit comprimé étant entièrement revêtu d'un film de matériau polymère insoluble dans des fluides aqueux, pellicule sur laquelle ont été pratiquées uniquement au laser une ou plusieurs incisions n'interférant pas avec le comprimé sous-jacent et délimitant une zone présentant une forme géométrique et des dimensions prédéterminées en fonction des temps de libération que l'on souhaite obtenir, ladite libération ayant lieu depuis la zone du noyau situé sous les surfaces de la pellicule protectrice délimitée par les incisions, lesquelles sont détachées lorsque le système thérapeutique vient en contact avec des fluides aqueux.

2. Système thérapeutique selon la revendication 1, dans lequel la pellicule protectrice n'est incisée qu'en coïncidant avec la première couche du comprimé (noyau).

3. Système thérapeutique selon la revendication 1, dans lequel la pellicule protectrice est incisée en coïncidant à la fois avec la première et la troisième couche.

4. Système thérapeutique selon la revendication 2, dans lequel la première et la troisième couche comprennent toutes les deux le même ingrédient actif.

5. Système thérapeutique selon la revendication 2, dans lequel la première et la troisième couche comprennent des ingrédients actifs différents.

6. Système thérapeutique selon la revendication 3, dans lequel la première et la troisième couche comprennent des ingrédients actifs différents.

7. Système thérapeutique selon la revendication 1, dans lequel la première et la troisième couche présentent une composition identique pour la libération contrôlée de l'ingrédient actif.

8. Système thérapeutique selon la revendication 1, dans lequel la première et la troisième couche présentent des compositions différentes pour la libération contrôlée de l'ingrédient actif.

9. Système thérapeutique selon la revendication 1, dans lequel la zone délimitée par l'incision (les incisions) sur la pellicule protectrice insoluble présente des dimensions comprises entre 2 et 50 % de la zone totale de la pellicule.

10. Système thérapeutique selon la revendication 9, dans lequel la zone délimitée par l'incision (les incisions) sur la pellicule protectrice insoluble présente des dimensions comprises entre 5 et 30 % de la zone totale de la pellicule.

11. Système thérapeutique selon la revendication 1, dans lequel la première couche comprend un ou plusieurs polymères capables de moduler la libération de l'ingrédient actif.

12. Système thérapeutique selon la revendication 11, dans lequel lesdits polymères constituent entre 1 % et 90 % en poids de ladite couche.

13. Système thérapeutique selon la revendication 12, dans lequel lesdits polymères constituent entre 5 % et 60 % en poids de ladite couche.

14. Système thérapeutique selon la revendication 1, dans lequel la première couche comprend des excipients capables d'accélérer la libération de l'ingrédient (des ingrédients) actif(s).

15. Système thérapeutique selon la revendication 14, dans lequel lesdits excipients sont des mélanges se désintégrant ou effervescents.

16. Système thérapeutique selon la revendication 1, dans lequel la troisième couche comprend un ou plusieurs polymères capables de moduler la libération de l'ingrédient (des ingrédients) actif (s).

17. Système thérapeutique selon la revendication 16, dans lequel lesdits polymères constituent entre 1 % et 90 % en poids de ladite couche.

18. Système thérapeutique selon la revendication 17, dans lequel lesdits polymères constituent entre 5 % et 60 % en poids de ladite couche.

19. Système thérapeutique selon la revendication 1, dans lequel la troisième couche comprend des excipients capables d'accélérer la libération de l'ingrédient actif.

20. Système thérapeutique selon la revendication 19, dans lequel lesdits excipients sont des mélanges se désagrégeant ou des effervescents.

21. Système thérapeutique selon la revendication 1, dans lequel la deuxième couche comprend un ou plusieurs polymères sélectionnés parmi des polymères pouvant être érodés de façon prédominante et des polymères pouvant être gélifiés de façon prédominante.

22. Système thérapeutique selon la revendication 21, dans lequel lesdits polymères constituent entre 5 et 90 % en poids de ladite couche.

23. Système thérapeutique selon la revendication 22, dans lequel lesdits polymères constituent 30 à 90 % en poids de ladite couche.

24. Système thérapeutique selon la revendication 1, dans lequel lesdites trois couches présentent chacune une épaisseur comprise entre 0,5 et 5 mm.

25. Système thérapeutique selon la revendication 1, dans lequel une seconde pellicule protectrice polymère gastrorésistante et entérosoluble est appliquée sur la pellicule protectrice polymère insoluble.

26. Processus de préparation du système thérapeutique selon la revendication 1, **caractérisé par le fait que** l'incision (les incisions) sur la pellicule protectrice sont exécutées au moyen d'un faisceau laser.

27. Processus selon la revendication 26, dans lequel l'incision (les incisions) sur la pellicule protectrice sont exécutées avec un dispositif laser à source CO₂ présentant une puissance de 20 W.
